# EUROPEAN PATENT SPECIFICATION

(11) **EP 2 684 601 B1**
(45) Date of publication and mention of the grant of the patent: **20.11.2019**
(21) Application number: 12005164.4
(22) Date of filing: 13.07.2012
(51) Int. Cl.: B01J 19/00, G01N 33/50, B01L 3/00, C12N 11/08

(54) **Formation of droplet or hydrogel arrays using hydrophilic-hydrophobic patterned surfaces for high-throughput screening applications**
Bildung von Tröpfchen- oder Hydrogelarrays unter Verwendung von hydrophil-hydrophob gemusterten Oberflächen für Abtastanwendungen mit hohem Durchsatz
Formation d'ensembles de gouttelettes ou hydrogel au moyen de surfaces à motifs hydrophiles-hydrophobes pour applications de dépistage à haut rendement

(43) Date of publication of application: 15.01.2014
(73) Proprietor: Karlsruher Institut für Technologie, 76131 Karlsruhe (DE)
(72) Inventor: Levkin, Pavel, Dr., 76344 Eggenstein-Leopoldshafen (DE); Boles, Erica, 76133 Karlsruhe (DE); Geyer, Florian, 69121 Heidelberg (DE)
(74) Representative: Müller-Boré & Partner Patentanwälte PartG mbB

(56) References cited:
- EP-A2- 1 364 702
- WO-A1-2006/083151
- WO-A1-2011/144743
- WO-A2-01/26797
- WO-A2-2012/078066
- US-A1- 2009 264 316
- FLORIAN L. GEYER ET AL: "Superhydrophobic-Superhydrophilic Micropatterning: Towards Genome-on-a-Chip Cell Microarrays", ANGEWANDTE CHEMIE INTERNATIONAL EDITION, vol. 50, no. 36, 12 July 2011 (2011-07-12) , pages 8424-8427, XP055045432, ISSN: 1433-7851, DOI: 10.1002/anie.201102545

## Description

The present invention relates to a method of forming an array of separated homogenous aqueous fluid microdroplets of a desired shape and size in a desired spatial pattern, comprising the steps of providing a patterned substrate with a solid support coated with a porous polymer layer or film comprising superhydrophilic areas having the desired shape and size, surrounded by superhydrophobic areas separating the hydrophilic areas into the desired spatial pattern, and applying the aqueous fluid to a multitude of the superhydrophilic areas at the same time.

High-throughput (HT) screening of live cells is an immensely important and growing task in areas ranging from studies of gene functions using RNA interference and the search for new drug candidates to screenings of new gene-delivery systems and the identification of factors controlling stem cell differentiation. Libraries containing thousands of nucleic acids, drug candidates, small molecules, or proteins are now available for HT cell screenings. During the last decade, cell microarrays - a miniaturized method for HT cell screening - have been developed.

However, due to cross-contamination of solutions and cells between neighboring spots on cell microarrays, currently most cell-based screenings are still performed using microplates, which commonly contain 96 or 384 individual wells to perform parallel cell experiments. Since the screening of thousands of molecules is often required in such experiments, HT cell-based screenings have become extremely expensive and unaffordable for many biological labs. Hundreds of microplates and expensive liquid-handling robotics are required for such experiments, which makes HT cell screening experiments accessible to only a few specialized screening centers worldwide. In addition to reducing the expense to perform a HT screen by using microplate-free technologies, such as cell microarrays, there is an increasing need to perform HT screens with non-adherent cells, cells in three-dimensional (3D) microenvironments, whole organisms, or with controlled diffusion of small molecules.

Currently, there are only a few methods available to screen non-adherent cells. As an example, a method has been developed to immobilize and pattern non-adherent cells on a surface by modifying glass with oleyl poly(ethylene glycol) ether, which incorporates into the cell membrane and acts as an anchor for cell attachment. Other methods using concave microwells, microfluidics-based cell culture platforms, or hanging droplet microplates have been developed for non-adherent cell culture. Since arrays of non-adherent cells such as some types of blood cells, stem cells, and cancer cells, as well as arrays of whole organisms can be helpful in answering certain biological questions, a method allowing HT screening of these cells and organisms is needed.

Many of the systems mentioned above such as cell microarrays and microplate-based assays require the use of liquid handling devices which can be very expensive, require many components, and are complicated to assemble and to operate.

In this context, EP 1 364 702 A2 describes a method for manufacturing an array plate for biomolecules and a method for manufacturing a biochip using said array plate: Further; US 2009/0264316 A1 describes methods for producing micropatterned surfaces by coating a surface with an organosilane and exposing said organosilane to UV/ozone treatment. Furthermore, WO 2012/078066 A2 describes a process for the deposition of biomaterials on surfaces involving the patterning of areas with different surface tensions. Finally, Geyer *et al.* (Geyer, F. L. et al.; Angew. Chem. Int. Ed., 50; 2011; pp. 8424-8427) describes methods for the preparation of arrays having a grid-like hydrophobic/hydrophilic pattern.

Accordingly, the technical problem underlying the present invention is to provide a method of forming arrays of separated homogenous fluid microdroplets or hydrogel patterns of a desired shape and size in a desired spatial pattern, as well as arrays of biological specimens that do not adhere to and/or do not grow on surfaces, without the use of expensive liquid-handling devices.

The solution to the above technical problem is achieved by the embodiments characterized in the claims.

In particular, the present invention relates to a method of forming an array of separated homogenous aqueous fluid microdroplets of a desired shape and size in a desired spatial pattern, comprising the steps of:
(a) providing a patterned substrate with a solid support coated with a porous polymer layer or film comprising
   (i) superhydrophilic areas having static, advancing and receding water contact angles (WCAs) of less than 20°, and having the desired shape and size; surrounded by
   (ii) hydrophobic areas having a WCA of greater than 130° and a width of 200 µm or less, and separating the hydrophilic areas into the desired spatial pattern;
      and
(b) applying the aqueous fluid to a multitude of the hydrophilic areas at the same time, wherein step (b) comprises spreading or rolling an amount of the aqueous fluid along the surface of the patterned substrate.

The term "aqueous fluid" as used herein is not particularly limited and relates to any aqueous fluids that might be of interest for the formation of a respective microdroplet array. In particular, the aqueous fluid can be selected from the group consisting of water, aqueous solutions and aqueous media. In a specific embodiment, the aqueous fluid is an aqueous solution that can form a hydrogel upon crosslinking, e.g. a solution containing one or more compounds which polymerize upon contact with a suitable crosslinker, or upon UV-initiated polymerization. In this specific embodiment, the aqueous fluid microdroplets form hydrogel micropads upon crosslinking.

In another embodiment, the aqueous fluid can contain biological specimens. These biological specimens can be selected from the group consisting of cells, bacteria, viruses and multicellular organisms. Preferably, said biological specimens do not adhere and/or grow on surfaces. The term "cell" as used in this context is not particularly limited and encompasses any kind of prokaryotic and eukaryotic cells that might be of interest. The term "multicellular organism" as used in this context is not particularly limited and encompasses any multicellular organisms in any developmental stage that might be of interest, provided that the multicellular organism is not too big to fit into the microdroplets formed. Multicellular organisms that are of particular interest in the context of the present invention are *Danio rerio, i.e.* zebrafish, e.g. zebrafish eggs or zebrafish embryos, and the nematode *Caenorhabditis elegans.*

The term "microdroplet" as used herein refers to the droplets formed in the method of the present invention. Patterns confining the microdroplets can have any desired shape, e.g. a circular or hexagonal shape, and can have also sharp-edged geometries such as triangular shapes. The patterns can have dimensions of 50 µm to 1 cm, and microdroplets can have volumes of 100 pi to 30 µl. The number of microdroplets formed in the method of the present invention is not particularly limited and depends on the size of the patterned substrate and the number of hydrophilic areas formed thereon. As an example, arrays, in particular high-density arrays, of thousands of microdroplets can be formed. The microdroplets are preferably homogenous, *i.e.* they all have the same size and shape, and they are separated, *i.e.* they are separated from each other by the hydrophobic areas separating the hydrophilic areas on which the microdroplets form. In this manner, the individual microdroplets are completely isolated. An array of microdroplets as formed in the method of the present invention is sometimes referred to as "DropletArray" hereinafter.

The term "spatial pattern" as used herein is not particularly limited and can encompass any desired pattern of microdroplets. Preferably, the pattern is chosen such that the density of microdroplets per area is maximized.

In the following, the terms "area" and "spot" will be used synonymously. As an example, the hydrophilic areas of the patterned substrates used in the present invention can be referred to as hydrophilic spots or just as spots.

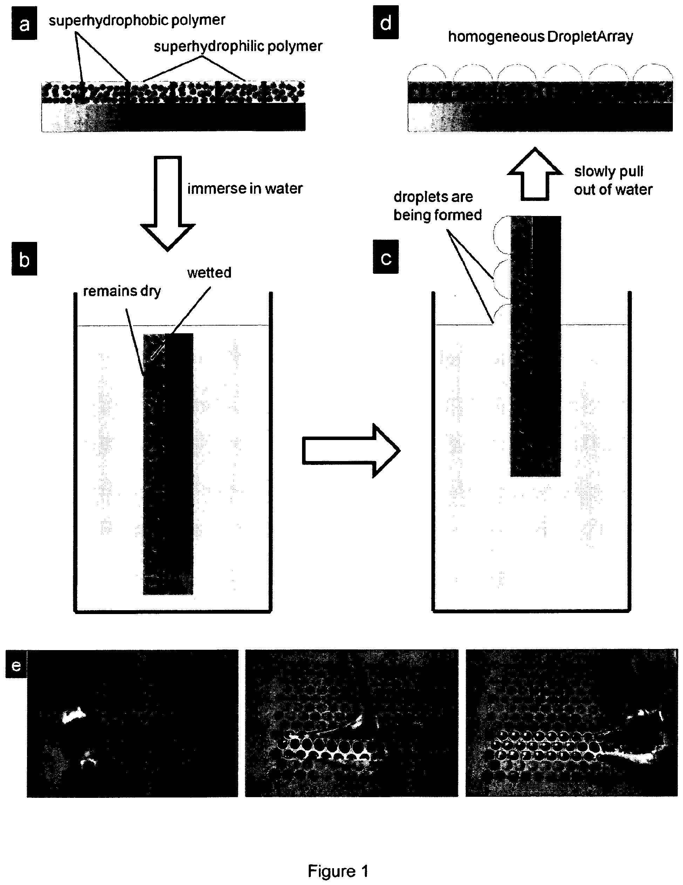

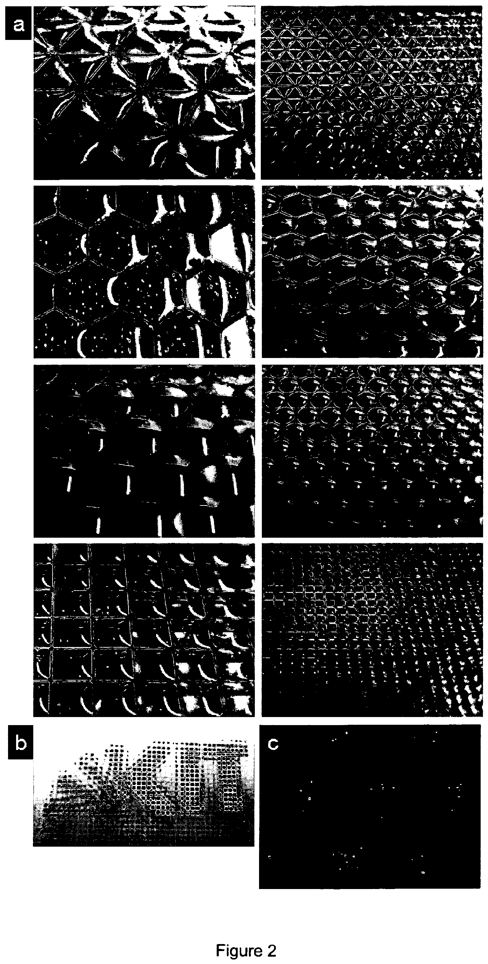

Step (b) of the method of the present invention, *i.e.* the step of applying the aqueous fluid to a multitude of the hydrophilic areas at the same time is a step wherein the aqueous fluid is applied at the same time to at least more than one, preferably more than 5, 10, 20, 50, 100, 500, or 1000 spots, more preferably to all spots. Alternatively, the aqueous fluid is applied at the same time to at least 5, 10, 15, 20, 30, 50, or 75% of all spots, more preferably to 100% of the spots. In particular, the aqueous fluid is not applied on a spot-by-spot basis. Preferably, the aqueous fluid is not applied with the help of an automated liquid-handling device. In a preferred embodiment, the aqueous fluid is applied manually, *i.e.* step (b) of the method of the present invention is performed manually. According to the present invention, step (b) comprises spreading or rolling an amount of the aqueous fluid along the surface of the patterned substrate. The aqueous fluid can be spread or rolled along the surface of the substrate e.g. with the help of a pipette. The amount of fluid used is preferably an amount that is just sufficient to form microdroplets on all hydrophilic spots of the patterned substrate. An illustration is shown in Fig. 1e. Further, images of DropletArrays formed with the method of the present invention are shown in Fig. 2.

The patterned substrate used in the method of the present invention is preferably a microarray, more preferably a cell microarray. Further, said patterned substrate preferably has a flat surface, and does not comprise any physical barriers, such as wells, recesses, indentations, depressions, ridges or the like, separating the individual hydrophilic areas from each other.

The porous polymer layer of film coated on the patterned substrate used in the method of the present invention is preferably biocompatible, *i.e.* it does not impair or negatively affect the growth, proliferation or well-being of any cells, bacteria or multicellular organisms that are trapped in the microdroplets formed, and does not impair the structural or functional integrity of any biologically active compounds, such as nucleic acids, peptides, proteins and enzymes, that may be present in the microdroplets.

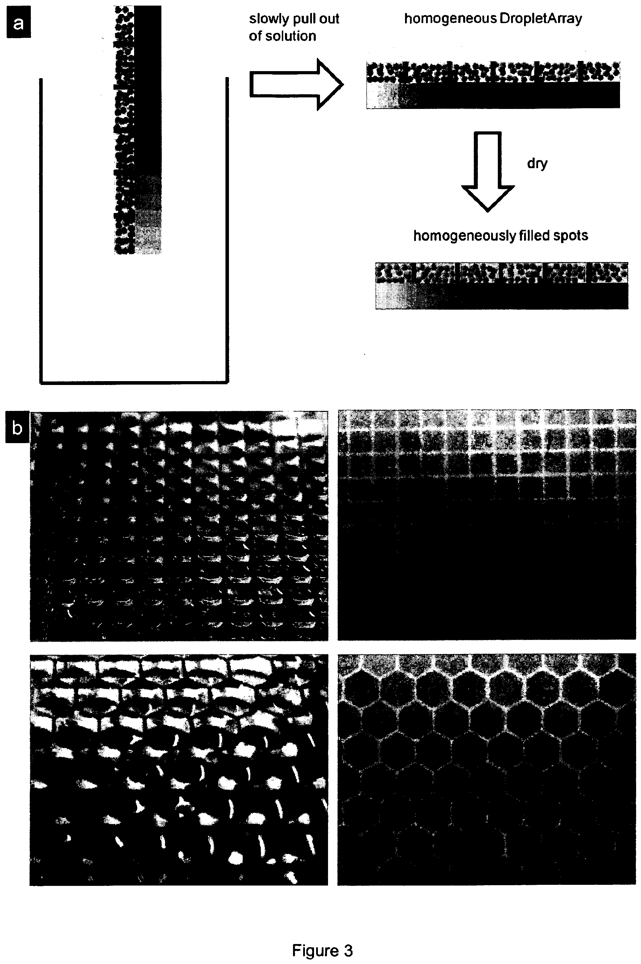

In preferred embodiments, the superhydrophilic areas in the patterned substrate used in the method of the present invention have at least one compound absorbed thereto which can diffuse into the aqueous fluid microdroplets formed. Preferably, the at least one compound is selected from the group consisting of chemical compounds, pharmaceutically active agents, biologically active agents, toxins, nucleic acids, peptides, proteins, enzymes, crosslinkers, buffer substances, water-soluble polymers and mixtures thereof. Methods for adsorbing any of said compounds to the hydrophilic areas are not particularly limited and include for example the formation of microdroplets of an aqueous fluid containing said compounds and subsequent drying of the microdroplets. A schematic overview of this method, exemplified with the deposition of the dye methyl green, is shown in Fig. 3.

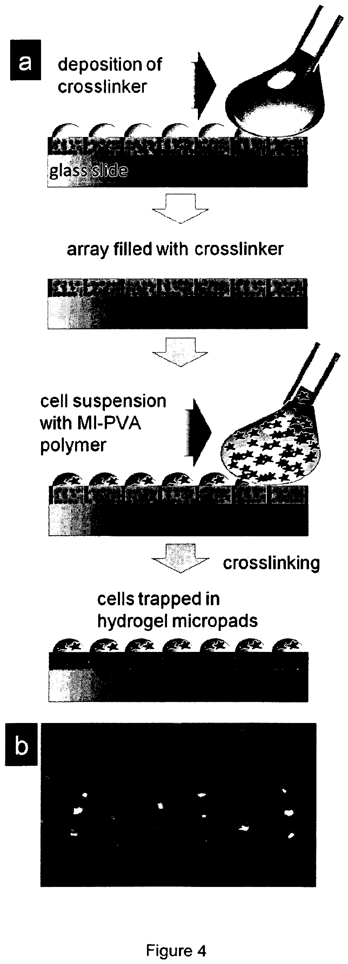

In a particular embodiment of the method of the present invention, an array of hydrogel micropads, e.g. containing cells that are immobilized in said hydrogel, can be formed. In this embodiment, the hydrophilic areas on the patterned substrate have a crosslinker adhered thereto, and the aqueous fluid is a solution that can form a hydrogel upon crosslinking, so that the aqueous fluid microdroplets form hydrogel micropads. A schematic representation of this embodiment is shown in Fig. 4. Suitable crosslinkers and gel-forming solutions are not particularly limited and are known in the art. As an example, 3-D Life Biomimetic Hydrogels for Three-Dimensional Cell Culture (Cellendes, Reutlingen, Germany) can be used, which are based on the use of maleimide-functionalized polymers and thiol-functionalized polyethylene glycol crosslinkers.

The patterned substrates used in the method of the present invention, as well as methods for producing the same, are preferably as described hereinafter.

In the following, the terms "hydrophilic" and "superwetting" will be used synonymously.

The superhydrophobic surfaces and barriers of the patterned substrate used in the present invention result in the prevention of proliferation and migration of cells. The use of such superhydrophobic barriers is an approach for controlling cell proliferation and migration. The combination of the watertight hydrophilic (superwettable) areas with the highly efficient cell-barriers represents a new approach for designing e.g. cell arrays and has the potential to push the spot-density towards its theoretical maximum that is only given by the necessary amount of cells in each spot. Stringent control over all important parameters (feature shape and size, absorbable volume, thickness of the polymer film, cell-migration, transparency) and the simple and inexpensive production enables an easy adjustment to any other application where cell-growth in a predetermined spatial order on a flat substrate is needed.

In general, surface properties can be classified into hydrophobic and hydrophilic surfaces depending on the value of the water contact angle (WCA). A surface having a WCA greater than 90° is referred to as hydrophobic, whereas a WCA smaller than 90° is referred to as hydrophilic. The maximum water contact angle on a smooth surface is about 120°. In practice, two types of WCA values are used: static and dynamic. Static water contact angles (qstat) are obtained by sessile drop measurements, where a drop is deposited on the surface and the value is obtained by a goniometer or a specialized software. Dynamic contact angles are non-equilibrium contact angles and are measured during the growth (advancing WCA qadv) and shrinkage (receding WCA qrec) of a water droplet. The difference between qadv and qrec is defined as contact angle hysteresis (CAH). According to the present invention, surfaces with high WCA, preferably greater than 130°, more preferably greater than 140°, most preferably greater than 150°, and/or low water CAH (less than about 30°, preferably less than about 20°, more preferably less than about 10°) are called superhydrophobic. Water droplets do not stick to such surfaces and simply roll off. According to the present invention, surfaces with both static, advancing and receding WCAs less than 20°, preferably less than 10°, more preferably less than 5°, most preferably close to or of 0° are called superhydrophilic. Water droplets are rapidly absorbed by such surfaces.

According to the present invention, the patterned substrate is a surface having superhydrophilic patterns on a superhydrophobic background. In a more preferred embodiment of the present invention, the patterned substrate is a surface having 6, 12, 24, 96, 384, 1536 superhydrophilic areas on a superhydrophobic background.

Superhydrophobic and superhydrophilic materials are known in the art. Within the scope of the present invention, the porous polymer layer which exhibits superhydrophobic properties can be comprised of a crosslinked polyvinyl monomer, wherein the polyvinyl monomer is one or more monomers selected from the group consisting of alkylene diacrylates, oligoethyleneoxide diacrylates or dimethacrylates, alkylene dimethacrylates, pentaerythritol tetraacrylate, pentaerythritol tetramethacrylate, trimethylopropane acrylate, trimethylopropane methacrylate, divinylbenzene, and divinylnaphthalene. In a preferred embodiment, the polyvinyl monomer is selected from the group consisting of ethylene dimethacrylate and divinylbenzene, whereof ethylene dimethacrylate is particularly preferred.

The superhydrophobic porous polymer may further comprise a monovinyl monomer, wherein the monovinyl monomer is selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, styrene, vinylnaphthalene, vinylanthracene, and derivatives thereof, wherein the alkyl group in each of the alkyl monomers has 1-18 carbon atoms. In a preferred embodiment, the monovinyl monomer is selected from the group consisting of methyl methacrylate, butyl methacrylate, benzyl methacrylate and styrene. The above polyvinyl monomer is copolymerized with the monovinyl monomer affording the porous superhydrophobic polymer layer.

According to the present invention, as the porous polymer layer exhibits superhydrophilic properties, the porous polymer layer can be comprised of a crosslinked polyvinyl monomer, wherein the polyvinyl monomer is one or more monomers selected from the group consisting of alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1-4 carbon atoms, oligoethylene glycol diacrylates, vinyl esters of polycarboxylic acids, pentaerythritol diacrylate, pentaerythritol triacrylate, pentaerythritol dimethacrylate, and pentaerythritol trimethacrylate. In a preferred embodiment, the polyvinyl monomer is selected from the group consisting of ethylene dimethacrylate and methylene-bis-acrylamide.

The superhydrophilic porous polymer may further comprise a monovinyl monomer, wherein the monovinyl monomer is selected from the group consisting of vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1-4 carbon atoms, hydroxyalkyl acrylates and acrylamides, hydroxyalkyl methacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1- propanesulfonic acid, 2-acrylamido-glycolic acid, [2-(methacryloyloxy)ethyl] trimethylammonium chloride, and N-[3-(dimethylamino)propyl] methacrylamide. In a preferred embodiment, the monovinyl monomer is selected from the group consisting of 2-hydroxyethyl methacrylate, decaethylene glycol methacrylate, N-isopropylacrylamide, and acrylamide. The above polyvinyl monomer is copolymerized with the monovinyl monomer affording the porous superhydrophilic polymer layer.

In a particularly preferred embodiment of the present invention, the porous polymer layer comprises at least one member selected from the group consisting of porous poly(butyl methacrylate-co-ethylene dimethacrylate) (BMAcoEDMA), porous poly(methyl methacrylate-co-ethylene dimethacrylate) (MMAcoEDMA), porous poly(2-hydroxyethyl methacrylate-co-ethylene dimethyl-acrylate) (HEMAcoEDMA), porous poly(styrene-co-1,4-divinylbenzene) (STcoDVB), porous poly(2,2,3,3,3-pentafluoropropyl methacrylate-co-ethylene dimethacrylate) (PFPMAcoEDMA).

In a preferred embodiment of the patterned substrate used in the present invention, the pattern is created by modifying the surface of the porous polymer layer using photografting through a photomask. In a more preferred embodiment of the present invention, the superhydrophobic areas are created by photografting the porous polymer layer being a porous superhydrophilic layer. In another preferred embodiment of the present invention, the superhydrophilic areas are created by photografting the porous polymer layer being a porous superhydrophobic layer.

In a preferred embodiment of the present invention, the patterned substrate is a microarray which preferably exhibits an array-pattern which is created by photografting the porous polymer layer using a photomask. The use of photochemical methods allows precisely controlling the geometry, size and distance between the spots by the design of the photomask.

According to the present invention, the porous polymer layer itself may be hydrophobic, preferably superhydrophobic. In this embodiment, the superhydrophobic barriers originate from the porous polymer layer, whereas the superhydrophilic spots/areas are created by modifying, preferably photografting the porous polymer layer. On the other hand, the porous polymer layer itself may be hydrophilic, preferably superhydrophilic. In such an embodiment, the superhydrophobic barriers are created by photografting the porous polymer layer.

According to the present invention, the superhydrophobic barriers are preferably created by modifying, preferably photografting the porous polymer layer being a porous (super)hydrophilic polymer layer. In a preferred embodiment of the present invention, the superhydrophobic barriers are obtained by UV-initiated photografting the porous polymer using a compound capable to impart a superhydrophobic functionality to the superhydrophilic porous polymer. In a preferred embodiment of the present invention, the superhydrophilic porous polymer layer is photografted using a photografting mixture containing one or more mono- or poly- vinyl monomers selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, for example 2,2,3,3,3-pentafluoropropyl methacrylate, styrene, vinylnaphthalene, vinylanthracene, alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1-4 carbon atoms, oligoethylene glycol diacrylates, vinyl esters of polycarboxylic acids, pentaerythritol diacrylate, pentaerythritol triacrylate, pentaerythritol dimethacrylate, and pentaerythritol trimethacrylate and derivatives thereof, wherein the alkyl group in each of the alkyl monomers has 1-18 carbon atoms. Particularly preferred is a photografting mixture consisting of 15wt.% 2,2,3,3,3-pentafluoropropyl methacrylate, 1wt.% ethylene dimethacrylate in 1/3 (v./v.) mixture of water - tert-butanol containing 0.25wt.% benzophenone.

According to the present invention, the superhydrophilic areas may be created by modifying, preferably photografting the porous polymer layer being a porous superhydrophobic polymer layer. In a preferred embodiment of the present invention, imparting superhydrophilic properties to the superhydrophobic porous polymer layer is accomplished using a hydrophilic compound, for example mono- or polyvinyl monomer selected from the group consisting of monovinyl monomer, wherein the monovinyl monomer is selected from the group consisting of vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1-4 carbon atoms, hydroxyalkyl acrylates and acrylamides, hydroxyalkyl methacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1- propanesulfonic acid, 2-acrylamido-glycolic acid, [2-(methacryloyloxy)ethyl]trimethylammonium chloride, N-[3-(dimethylamino)propyl] methacrylamide or alkylene diacrylates, olygoethyleneoxide diacrylates or dimethacrylates, alkylene dimethacrylates, pentaerythritol tetraacrylate, pentaerythritol tetramethacrylate. Preferably, said hydrophilic compound is a photografting mixture comprising [2-(methacryloyloxy)ethyl]-trimethylammonium chloride (META) or 2-acryloamido-2-methyl-1-propanesulfonic acid. According to a more preferred embodiment, the hydrophilic photografting mixture consists of 15wt.% META, and 0.25wt.% benzophenone dissolved in a 1:3 (v/v) mixture of water and tert-butanol.

In the thus obtained pattern, an easy and homogeneous adsorption of the spotting solution in the porous superhydrophilic areas can be guaranteed. Each area is separated from adjacent areas with superhydrophobic barriers that render the areas impermeable for all water based solutions.

In a preferred embodiment of the present invention, the superhydrophilic areas of the patterned substrate are separated by the superhydrophobic areas so as to form an array of superhydrophilic areas surrounded by a superhydrophobic porous polymer surface.

In another preferred embodiment of patterned substance according to the present invention, the hydrophilic areas are separated by the superhydrophobic areas of porous polymer surface so as to form superhydrophilic channels. In a more preferred embodiment of the present invention, the patterned substance comprises superhydrophilic channels on a superhydrophobic background and the patterned substance is used for microfluidic applications.

In another preferred embodiment of the present invention, the superhydrophilic areas which are separated by the superhydrophobic barriers in the patterned substrate have a square shape. Superhydrophilic areas having a square shape allow dense packing of the areas and ease the readout. Nevertheless, the patterned substrate according to the present invention is not limited to any array-format, but represents a more general and novel approach that allows patterning and for example culturing of cells in a predesigned spatial order.

In one embodiment of the present invention, the area-density of the superhydrophilic areas is increased. Preferably, the amount of superhydrophilic areas is at least 200 per cm² of the patterned substrate, more preferably at least 300 per cm² of the patterned substrate, even more preferably at least 400 per cm² of the patterned substrate. In a particularly preferred embodiment of the present invention, the amount of superhydrophilic areas is at least 500 per cm² of the patterned substrate.

According to an even more preferred embodiment of the present invention, the patterned substrate used in the present invention has a size which fits on a standard microtiter scaled plate (12 x 8 cm), preferably a size of about 11 x 7 cm with at least 40000, preferably at least 50000 hydrophilic areas separated by superhydrophobic background. In another preferred embodiment of the present invention, the patterned substrate used in the present invention is a microslide of about 1 x 7 cm.

Preferably, in the patterned substrate used in the present invention, the overall area-number on a single chip is significantly increased. When used as a cell microarray, minimal area of a single area in the patterned substrate according to the present invention is limited by the amount of cells needed in each area to obtain statistically valid data. In a particularly preferred embodiment of the present invention, each of the superhydrophilic areas defined in (i) has a side length of 1000 µm or less, preferably 600 µm or less, more preferably 400 µm or less, and most preferably 335 µm or less, particularly under the provision that the superhydrophilic areas have a square shape. In another preferred embodiment of the present invention, the size of the superhydrophilic areas is large enough to accommodate at least 100 cells, preferably 200 cells, more preferably at least 300 cells.

According to the present invention, the superhydrophobic barriers have a width of 200 µm or less, more preferably 100 µm or less and even more preferably 60 µm or less. A significant advantage of the patterned substrate according to the present invention is that the superhydrophobic barriers completely prevent cross-contamination between the areas, despite the reduced width of the barriers.

Further, according to the present invention, the thickness of the porous polymer layer is not limited. Regarding transparency properties or reducing manufacturing costs, a thin porous polymer layer is preferred. Particularly preferred is a porous polymer layer having a thickness of 45 µm or less, preferably 30 µm or less, and more preferably 15 µm or less.

The patterned substrate used in the present invention preferably further comprises (iii) at least one biologically active agent provided in at least one superhydrophilic area. According to a preferred embodiment, each superhydrophilic area of the patterned substrate is provided with at least one biologically active agent. In this embodiment, the superhydrophobic barriers not only prevent cross-contamination of any aqueous liquids between the areas, but also prevent proliferation and migration of cells between hydrophilic areas. That is, superhydrophobic barriers between the superhydrophilic areas of the patterned substrate according to the present invention stop cell proliferation and migration from one area to another. With this technique, a patterned substrate can be designed with an area-density that is not achievable with common methods.

The very good wetting property of the porous polymer surface inside hydrophilic areas guarantees an easy and homogeneous spreading of the solutions inside the areas independent of their geometry. In addition, the superhydrophobic barriers prevent cells from migrating between the adjacent areas. Thus, the serious problem of cell-migration between the areas known for cell arrays of the prior art can be solved using the patterned substrate as described herein.

The patterned substrate used in the present invention can be manufactured by a method, comprising the steps of:
(a) providing a polymerization mixture between two solid supports;
(b) polymerizing the polymerization mixture to form a porous polymer layer;
(c) removing one of the two solid supports; and
(d) modifying the surface of the porous polymer layer, thereby creating a pattern of hydrophilic areas surrounded by hydrophobic areas.

The solid support may be of any material known in the art which is solid, preferably at a temperature within the range of -30°C to 130°C, more preferably within the range of 0°C to 40°C, more preferably at room temperature. The solid support can be selected from the group consisting of glass, metal, such as a stainless steel plate, or an aluminum foil, plastic, concrete, wood, and masonry. Preferably, the solid support is transparent, wherein a glass solid support is particularly preferred. Such a glass solid support may be activated and/or modified prior to use.

In order to achieve covalent attachment of the porous polymer layer, the glass surface is preferably first activated, and then functionalized with an anchor-group. For example, cleaned glass plates may be immersed in 1 M NaOH for one hour and afterwards washed with deionized water followed by immersing them in 1 M HCl for 30 min, washing with deionized water and drying with a nitrogen gun. Further, the glass solid support may be modified by for example dropping several drops of a solution containing 20% 3-(trimethoxysilyl)propyl methacrylate in ethanol (adjusted to pH = 5 with acetic acid) on an activated glass plate, covering the glass plate with another activated glass plate, thereby ensuring that no bubbles are trapped between the two glass plates, reapplying said solution after 30 minutes and after another 30 minutes washing the glass plates with acetone and drying with a nitrogen gun.

The glass solid supports may be made inert for example by fluorination using for example tridecafluor-(1,1,2,2)-tetrahydrooctyltrichlorosilane placed in a vacuumed desiccator together with the glass solid support for 2h.

In step (a) of the method for the manufacture of a patterned substrate, a polymerization mixture is provided between two solid supports. The solid supports may be made of the same material, or of different materials. In particular, the aforementioned materials suitable for the patterned substrate according to the present invention are preferred. Preferably, the solid supports are made of a glass material, of which one or both may be modified and/or activated prior to use, such as a glass modified with anchor groups. Preferably, the solid support is a solid support as defined herein.

In step (b) of the method for the manufacture of a patterned substrate, polymerization is carried out in the mold between the two solid supports. The polymerization may involve a free radical polymerization of a polyvinyl monomer and a monovinyl monomer in the presence of porogenic solvents. The free radical initiation is preferably done either thermally or by irradiation with UV- or visible light or γ-irradiation. When polymerization is carried out by thermal initiation, the thermal initiator is generally a peroxide, a hydroperoxide, or an azo-compound selected from the group consisting of benzoyl peroxide, potassium peroxodisulfate, ammonium peroxodisulfate, t-butyl hydroperoxide, 2,2'-azobisiobutyronitrile (AIBN), and azobisisocyanobutyric acid, and the thermally induced polymerization is performed by heating the polymerization mixture to temperatures between 30°C and 120°C. Polymerization can also be achieved using photoinitiators including, but not limited to, benzophenone, 2,2-dimethoxy-2-phenylaceto-phenone, dimethoxyacetophenone, xanthone, thio-xanthone, camphorquinone their derivatives, and mixtures thereof.

The polymerizing mixture is not limited, as long as said polymerizing mixture affords a porous polymer layer having superhydrophobic or superhydrophilic properties. Depending on the aimed surface property of the porous polymer, i.e. hydrophilicity or hydrophobicity, the respective monomers are selected accordingly.

Preferably, the porous polymer layer possesses bulk hydrophilicity and is prepared by radical polymerization of a mixture containing a polyvinyl crosslinker and monovinyl monomers in the presence of an inert porogen.

The porogen (i.e. liquids that cause formation of the porous structure of the polymer) used to prepare the porous polymer layer may be selected from a variety of different types of compounds. For example, suitable liquid porogens include aliphatic hydro-carbons, aromatic hydrocarbons, esters, amides, alcohols, ketones, ethers, solutions of soluble polymers, and mixtures thereof. For preparation of superhydrophilic materials, water may also be used. The porogen is generally present in the polymerization mixture in an amount of from about 40 to 90 vol%, more preferably from about 50 to 80 vol%. In a preferred embodiment, the porogen is a mixture of 1- decanol and cyclohexanol.

Preferably, the polyvinyl crosslinker is selected from the group consisting of alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1 - 4 carbon atoms, oligoethylene glycol diacrylates, vinyl esthers of polycarboxylic acids, pentaerythritol diacrylate, pentaerythritol triacrylate, pentaerythritol dimethacrylate, and pentaerythritol trimethacrylate.

Preferably, the monovinyl monomers are selected from the group consisting of alkyl vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1 - 4 carbon atoms, hydroxyalkyl acrylates and acrylamides, hydroxymethacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1-propanesulfonic acid, 2-acrylamidoglycolic acid, [2-(methacrylooxy)ethyl] trimethylammonium chloride, and N-[3-(dimethylamino)propyl]methacrylmide.

Preferably, the inert porogen is selected from the group consisting of aliphatic hydrocarbons, aromatic hydrocarbons, esters, amides, alcohols, ketones, ethers, solutions of soluble polymer, water, and mixtures thereof.

Preferably, the porous polymer layer comprises at least one member selected from the group consisting of porous poly(butyl methacrylate-co-ethylene dimethacrylate) (BMAcoEDMA), porous poly(methyl methacrylate-co-ethylene dimethacrylate) (MMAcoEDMA), porous poly(2-hydroxyethyl methacrylate-co-ethylene dimethyl-acrylate) (HEMAcoEDMA), porous poly(styrene-co-1,4-divinylbenzene) (STcoDVB), porous poly(2,2,3,3,3-pentafluoropropyl methacrylate-co-ethylene dimethacrylate) (PFPMAcoEDMA).

More preferably, the hydrophilic porous polymer layer is made of poly(2-hydroxyethyl methacrylate-co-ethylene dimethacrylate) synthesized by copolymerization of 2-hydroxyethyl methacrylate (HEMA) and ethylene dimethacrylate (EDMA) in the presence of porogens (as shown for example in Fig. 1).

Preferably, the porous polymer layer is made of poly(butyl methacrylate-co-ethylene dimethyl-acrylate) (BMAcoEDMA). The porous polymer layer of BMAcoEDMA is synthesized by copolymerization of butyl methacrylate (BMA) and ethylene dimethacrylate (EDMA) in the presence of porogens.

In the embodiment that the porous polymer layer should exhibit superhydrophobic properties, the polyvinyl monomer is one or more monomers selected from the group consisting of alkylene diacrylates, alkylene dimethacrylates, penta-erythritol tetraacrylate, pentaerythritol tetramethacrylate, trimethylopropane acrylate, trimethylopropane methacrylate, divinylbenzene, and divinyl-naphthalene. Preferably, the polyvinyl monomer is selected from the group consisting of ethylene dimethacrylate and divinylbenzene, whereof ethylene dimethacrylate is particularly preferred.

Further, the superhydrophobic porous polymer further comprises a monovinyl monomer, wherein the monovinyl monomer is selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, styrene, vinylnaphthalene, vinylanthracene, and derivatives thereof, wherein the alkyl group in each of the alkyl monomers has 1-18 carbon atoms. Preferably, the monovinyl monomer is selected from the group consisting of methyl methacrylate, butyl methacrylate, benzyl methacrylate and styrene. The above polyvinyl monomer is copolymerized with the monovinyl monomer in the presence of a porogenic solvent affording the porous superhydrophobic polymer layer.

In the embodiment that the porous polymer layer should exhibit superhydrophilic properties, the porous polymer layer can be comprised of a crosslinked polyvinyl monomer, wherein the polyvinyl monomer is one or more monomers selected from the group consisting of alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1-4 carbon atoms, oligoethylene glycol diacrylates, vinyl esters of polycarboxylic acids, pentaerythritol diacrylate, pentaerythritol triacrylate, pentaerythritol dimethacrylate, and pentaerythritol trimethacrylate. Preferably, the polyvinyl monomer is selected from the group consisting of ethylene dimethacrylate and methylene-bis-acrylamide.

The superhydrophilic porous polymer further comprises a monovinyl monomer, wherein the monovinyl monomer is selected from the group consisting of vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1-4 carbon atoms, hydroxyalkyl acrylates and acrylamides, hydroxyalkyl methacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1-propanesulfonic acid, 2-acrylamido-glycolic acid, [2-(methacryloyloxy)ethyl]trimethylammonium chloride, and N-[3-(dimethyl-amino)propyl] methacrylamide. Preferably, the monovinyl monomer is selected from the group consisting of 2-hydroxyethyl methacrylate, decaethylene glycol methacrylate, N-isopropylacrylamide, and acrylamide. The above polyvinyl monomer is copolymerized with the monovinyl monomer in the presence of a porogenic solvent affording the porous superhydrophilic polymer layer.

The ratio of polyvinyl monomer to monovinyl monomer is not limited as long as the obtained porous polymer exhibits superhydrophobic properties or superhydrophilic properties. In a preferred embodiment, the polyvinyl monomer content is within the range of 5 to 30wt.%, preferably 10 to 25wt.%, more preferably 15 to 20wt.%, based on the total amount of the polymerization mixture. The monovinyl monomer content is preferably within the range of 10 to 50wt.%, preferably 15 to 45wt.%, more preferably 20 to 40wt.%, based on the total amount of the polymerization mixture.

Particularly preferred polymerization mixtures include:
(i) poly(2-hydroxyethyl methacrylate-co-ethylene dimethacrylate) (HEMA-co-EDMA): 2-hydroxyethyl methacrylate (HEMA) (24wt.%), ethylene dimethacrylate (EDMA) (16wt.%), 1-decanol (12wt.%), Cyclohexanol (48wt.%) and 2,2-dimethoxy-2-phenylaceto-phenone (DMPAP) (1wt.% with respect to monomers),
(ii) poly(2-hydroxyethyl methacrylate-co-ethylene dimethacrylate): 2-hydroxyethyl methacrylate (24wt.%), ethylene dimethacrylate (16wt.%), 1-decanol (40wt.%), cyclohexanol (20wt.%) and 2,2-dimethoxy-2-phenylacetophenone (1wt.% with respect to monomers), and
(iii) poly(butyl methacrylate-co-ethylene dimethacrylate) (BMAcoEDMA): butyl meth-acrylate (BMA) (24wt.%), ethylene dimethacrylate (16wt.%), 1-decanol (40wt.%), cyclohexanol (20wt.%) and 2,2-dimethoxy-2-phenylacetophenone (1wt.% with respect to monomers).

After completion of the polymerization, one of the two substrates is removed in step (c).

In step (d) of the method for the manufacture of a patterned substrate, the surface of the porous polymer layer is modified, thereby creating a pattern of hydrophilic areas surrounded by hydrophobic areas. Preferably, the modification is carried out as described herein, most preferably by photografting including the various embodiments for photografting as described herein.

Preferably, the hydrophobic pattern is produced by photografting of the hydrophilic porous polymer layer with hydrophobic mono- or poly-vinyl monomers or mixtures thereof.

Preferably, the hydrophobic pattern is produced by photografting of the hydrophilic porous polymer layer with hydrophobic mono- or poly-vinyl monomers or mixtures thereof and the monovinyl monomers are selected from the group consisting of alkyl acrylates, alkyl methacrylates, aryl acrylates, aryl methacrylates, aryl alkyl acrylates, aryl alkyl methacrylates, fluorinated alkyl acrylates, fully or partially fluorinated alkyl methacrylates, for example 2,2,3,3,3-pentafluoropropyl methacrylate, styrene, vinylnaphthalene or vinylanthracene and derivatives thereof, wherein the alkyl group in each of the alkyl monomers has 1-20 carbon atoms.

More preferably, the hydrophobic pattern is produced by photografting of the hydrophilic porous polymer layer with hydrophobic mono- or poly-vinyl monomers or mixtures thereof and the polyvinyl monomers are selected from the group consisting of alkylene diacrylates, alkylene dimethacrylates, pentaerythritol tetraacrylates, pentaerythritol tetramethacrylates, trimethylopropane acrylates, trimethylopropane methacrylates, divinylbenzenes and divinylnaphthalenes.

Preferably, the modification of the surface of the porous layer in step (d) of the method for the manufacture of a patterned substrate is carried out by photografting the surface of the porous polymer layer using a photomask, wherein a photografting mixture is provided on the surface of the porous polymer layer.

Said photografting creates a pattern of superhydrophobic barriers separating superhydrophilic areas. As described earlier, the photomask may be applied so as to create the pattern, or to create the areas. Depending on the nature of the porous polymer layer (superhydrophilic or superhydrophobic), the photografting mixture applied to the porous polymer layer may induce either superhydrophobic or superhydrophilic regions on the porous polymer layer.

The polymerization of the polymerizing mixture may afford a superhydrophilic polymer layer which is subsequently photografted with a compound capable to impart superhydrophobicity to the porous polymer layer using a photomask. In this embodiment, the superhydrophilic areas originate from the regions of the porous polymer layer which have not been photografted. In addition, the regions which are photografted are transformed into superhydrophobic barriers separating the superhydrophilic areas.

Preferably, the porous polymer layer is a HEMAcoEDMA polymer layer, and the superhydrophobic barriers are created by photografting the HEMAcoEDMA surface with a photografting mixture. Particularly preferred is a photografting mixture containing 2-,2-,3-,3-,3-pentafluoropropyl methacrylate (PFPMA). In a more preferred embodiment of the present invention, the hydrophilic porous polymer surface comprises poly(2-hydroxyethyl methacrylate-co-ethylene dimethyl-acrylate) and the photografted hydrophobic monomer is 2,2,3,3,3-pentafluoropropyl methacrylate.

The photografting mixture may consists of 15wt.% of 2,2,3,3,3-pentafluoropropyl methacrylate and 1wt.% of ethylene dimethacrylate in 1/3 (v./v.) mixture of water - tert-butanol containing 0.25wt.% benzophenone.

Preferably, the hydrophilic porous polymer surface comprises HEMAcoEDMA and the photografted hydrophobic monomer is PFPMA. More preferably, a 12.5 µm-thin hydrophilic porous HEMAcoEDMA layer is patterned with PFPMA to form an array of square hydrophilic HEMAcoEDMA areas separated by hydrophobic areas.

The preparation of a patterned substrate ready for further applications such as printing biomolecules takes preferably about one hour.

The method for the manufacture of a patterned substrate described herein preferably further comprises the step (e) providing at least one biologically active agent in at least one superhydrophilic area, more preferably in each of the superhydrophilic areas.

In particular embodiments, the patterned substrates used in the method of the present invention, as well as methods for producing the same, are as described in European patent application 10 015 106.7 and/or as described in Geyer, F. L. et al., Superhydrophobic-Superhydrophilic Micropatterning: Towards Genome-on-a-Chip Cell Microarrays, Angewandte Chemie International Edition 2011, 50, pp. 8424-8427.

In the present application, a facile single-step method for creating thousands of isolated microdroplets with defined geometry and size, so-called DropletArrays, is described. It has been shown that thousands of superhydrophilic spots on a superhydrophobic background can be filled with an aqueous solution in a single step leading to the formation of high-density arrays of separated microdroplets. Bioactive molecules, non-adherent cells, or other organisms can be trapped in the completely isolated microdroplets for use in a variety of screening applications. Further, the preparation of a high-density array of hydrogel micropads incorporating live cells, which can be used for HT screening of cells in 3D microenvironments has been shown.

The present invention solves the problem of forming arrays of separated droplets or hydrogel pads in a desired pattern or shape in one easy step using superhydrophilic-superhydrophobic patterned surfaces. To form separated, homogeneous droplets or hydrogel pads, a droplet can be rolled along the surface. The droplets formed on the surface are due to the contrast in wettability of the superhydrophilic-superhydrophobic pattern. This invention allows thousands (or more) spots to be filled at once without the use of expensive liquid handling devices such as contact printers, pipetting robots, acoustic droplet ejection devices, etc. In addition, water-soluble chemicals or biological specimens smaller than the droplet size (e.g. cells, bacteria, zebrafish, *C. elegans,* etc.) can be encapsulated in droplets or hydrogels and deposited on the hydrophilic spots of the array. This invention solves the problem of patterning biological specimens that do not adhere to or do not grow on surfaces (e.g. non-adherent cells) which can be further used for high-throughput screening applications. This invention eliminates the need for physical barriers to separate samples in different spots such as when using microtiter plates and allows high-density arrays to be created. For use in chemical or drug screening applications, the spots can be pre-filled with a chemical of interest and will then diffuse into the formed droplets. Many of the current devices using the "hanging droplet" method to culture cells or tissues require liquid handling robots to form the droplets and are only available in the standard well-plate formats. The present invention allows the user to create droplets of various shapes and sizes simply by forming a different hydrophilic-hydrophobic pattern. In addition, the current hanging droplet devices only allow the droplets to be cultured upside down. With the present invention, the droplet can be hanging or upright and allows for imaging with either an upright or inverted microscope.

In conclusion, an easy method has been developed to fabricate arrays of separated droplets with virtually any geometry on a superhydrophilic, porous polymer patterned with a superhydrophobic background. The droplets can contain any substance dissolved or suspended in water, and drying the droplets results in the homogeneous deposition of the substance within the porous structure of the hydrophilic spots. This deposition method can be used to preprint an entire array with a crosslinker to form a hydrogel, composed of a crosslinkable polymer, encapsulating a cell suspension. Thus, non-adherent cells or other biological species (amoeba, bacteria, yeast) could easily be immobilized in a high density array and used for biological screenings. In combination with automated printing techniques, this method will enable the HT screening of many classes of biologically active compounds with respect to their influence on a broad variety of living specimens. In a similar but manual approach, the DropletArray can be used to study higher life-forms such as zebrafish eggs or embryos or worms (*C. elegans*). Due to the size of the specimens and the limits of current technology, these larger organisms may have to be deposited manually. However, the small droplet volume significantly decreases the required amount of substances. It also decreases the mobility of the organism, and in combination with the strict and ordered array pattern, it greatly simplifies observation of the sample. If fixed in a hydrogel, the movement of the organism can be completely immobilized, which allows for microscopic investigation of single compartments over a longer period of time. Not only can biologically active substances including many genes, drugs, proteins, and toxins unfold their biological effect on the cellular scale, but also on the scale of a whole organism. This DropletArray technique is envisioned to be an important step in the development of HT screening platforms for the investigation of gene functions and screening of small molecule libraries with the advantage of being compatible with non-adherent cell lines and multi-cellular biological species, up to whole organisms such as zebrafish and *C. elegans.*

The figures show:
Figure 1: Preparation of an array of microdroplets
   a) A patterned substrate in the dry state. A superhydrophilic, porous polymer layer is grafted with superhydrophobic moieties. b) When the hydrophobicity-patterned substrate is immersed in water, the hydrophilic areas become easily wetted while the hydrophobic areas remain in a dry Cassie-Baxter state. c) When slowly pulled out of water, only the hydrophilic areas remain filled with water and distinct droplets are formed. d) A homogeneous array of microdroplets is obtained. e) Spreading a droplet along the superhydrophilic-superhydrophobic patterned surface (1 mm diameter circles, 1.1 mm spot-to-spot spacing) forms droplets only in the hydrophilic spots. Figure 1e is according to the claimed invention.
Figure 2: Images of various arrays of microdroplets
   a) Images of arrays of microdroplets formed via the method of the present invention on superhydrophilic-superhydrophobic micropatterns with different geometries. b) A wetted microarray becomes transparent and displays a lens effect, showing the underlying paper printed with a logo. c) Fluorescent microscope image showing fluorescent MTly-CMV-eGFP-neo cells cultured in individual microdroplets on the array.
Figure 3: Deposition of compounds
   Deposition of a methyl green dye solution on a microarray is shown. a) Schematic representation of the droplet deposition method. The substrate is immersed in an aqueous solution, similar as described in Figure 1. b) Images of microdroplets formed from a methyl green solution (left) and then dried (right).
Figure 4: Formation of arrays of hydrogel micropads
   a) PEG-Link is deposited in the hydrophilic spots using the method of the present invention and allowed to dry. Then, a cell suspension mixed with MI-PVA is deposited using said method and crosslinking occurs to form cells encapsulated in hydrogel micropads. b) Image of hydrogel micropads stained with Rhodamine 6G in air.

The present invention will now be further illustrated in the following examples without being limited thereto.

### Examples

### Experimental procedures:

### General.

All polymerizations and photografting were carried out on an OAI Model 30 deep-UV collimated light source (San Jose, CA, USA) fitted with an USHIO 500 W Hg-xenon lamp (Japan). Irradiation intensity was calibrated to 12 mW/cm² (5.10 mW/cm² after cover glass slide, 4.77 mW/cm² after cover glass slide and photomask) using an OAI 360 UV power meter with a 260 nm probe head. Schott (Germany) Nexterion Glass B UV transparent glass plates were used as substrates for polymer layers. Monomers were purchased from Sigma-Aldrich. Biochemicals were purchased from Invitrogen. Cell lines and zebrafish were provided by the Institute of Toxicology and Genetics at Karlsruhe Institute of Technology. Hydrogels were made using the 3-D Life PVA-PEG Hydrogel Kit from Cellendes GmbH.

### Array preparation - Glass Surface Modification.

To achieve covalent attachment of the polymer layer, the glass surfaces are first activated and then functionalized with an anchor group for methacrylates.

Activation of glass slides: Clean glass slides are immersed in 1 M NaOH for 1 h and afterwards washed with deionized water, and then immersed in 1 M HCI for 30 min, washed with deionized water, and dried with a nitrogen gun.

Modification of glass slides: Several drops of a solution containing 20 %vol. 3-(trimethoxysilyl)propyl methacrylate in ethanol, adjusted to pH 5 with acetic acid, are dropped on an activated glass slide. The plate is covered with another activated glass slide. No bubbles should be trapped between the two slides. The solution is reapplied after 30 min. After another 30 min, the slides are washed with acetone and dried with a nitrogen gun.

Fluorination of glass slides: An activated glass slide is placed in a vacuumed desiccator together with a vial containing several drops of tridecafluoro-(1,1,2,2)-tetrahydrooctyltrichlorosilane over night.

### Array preparation - Polymerization Procedure and Photografting.

Polymerization: Two strips of Teflon film (American Durafilm Co.), defining the thickness of the polymer layer, are placed at the edges of a fluorinated glass slide and a modified glass slide is clamped on top of it. The polymerization mixture is injected in between the mold and irradiated for 15 min at 12 mW/cm² with a 260 nm UV light. The mold is then carefully opened using a scalpel.

An inert, fluorinated glass slide is used as the bottom plate. The inability of covalent attachment of the growing polymer to the fluorinated glass slide allows for the whole polymer to stick to the top, modified glass slide during the separation process. The fluorinated glass slide can be reused several times. The resulting nonporous superficial layer can be easily removed by applying and rapidly removing adhesive film (Tesa tape) immediately after separating the plates while the layer is still wetted with porogen. A homogeneous, porous surface is formed. The plate is washed extensively with methanol and kept in methanol for several hours to remove unreacted monomers and porogens.

Polymerization mixture: 2-hydroxyethyl methacrylate (24 wt %), ethylene dimethacrylate (16 wt %), 1-decanol (12 wt %), cyclohexanol (48 wt %), and 2,2-dimethoxy-2-phenylacetophenone (1 wt % with respect to monomers).

Photografting: The polymer layer is wetted with the photografting mixture and covered with a fluorinated glass slide. A photomask is placed on top and it is irradiated for 30 min with 12 mW/cm² 260 nm UV light. The obtained pattern is washed extensively with methanol and stored in methanol for several hours to remove excess monomer and porogen, and for sterilization before cell culture.

Photografting mixture: 2,2,3,3,3-pentafluoropropyl methacrylate (20 wt %), ethylene dimethacrylate (1.3 wt %), 1:3 (v/v) mixture of water:tert-butanol (78 wt %), benzophenone as the initiator (0.33 wt %).

### Hydrogel Array Protocol.

Hydrogel arrays were prepared using the Cellendes 3-D Life PVA-PEG Hydrogel Kit. For hydrogel visualization, the crosslinker solution was prepared by mixing 23 µL PEG-Link with 2 µL of 1 mg/mL 7-Diethylamino-3- (4-maleimidophenyl)-4-methylcoumarin in DMSO. Otherwise, just 25 µL of PEG-Link were used. The patterned substrate was placed in a Petri dish and a pipette was used to roll the 25 µL of crosslinker solution across the patterned substrate. Only the hydrophilic spots should be wetted with the crosslinker solution. Then, the crosslinker solution was allowed to dry. The maleimide polyvinyl alcohol (MI-PVA) cell solution was prepared such that the final concentration of MI was 6 mM. For a 1000 µm diameter, 1500 µm spot-to-spot spacing circular pattern, the following ingredients were used: Water: 12.5 µL; 10X CB pH 5.5 buffer: 2.5 µL; MI-PVA (30 mM MI stock): 5 µL; Cell suspension (16,000 cells/µL) in pH 5 medium: 5 µL of 80x10⁶ cells/mL cell suspension.

For application of the MI-PVA-cell solution a pipette was used to roll the 25 µL of the solution across the patterned substrate where the crosslinker solution was printed. This was done quickly and under humid conditions to prevent the gel from drying out. Droplets of medium were put around the substrate and put in the incubator until the polymerization had finished (about 5 min). Then, the hydrogel-cell array was immersed in medium.

### Example 1:

### Formation of DropletArravs

To make an array of superhydrophilic spots on a superhydrophobic surface, a 12.5 µm thin, superhydrophilic film of nanoporous poly(hydroxyethyl methacrylate-co-ethylene dimethacrylate) (HEMA-EDMA) was photografted through a quartz photomask with pentafluoropropyl methacrylate (PFPMA) to create superhydrophobic regions (cf. Experimental procedures). Using this method, arrays of superhydrophilic spots with specific geometry and size can be created. It has been shown that it is possible to create arrays of thousands of identical microdroplets in a single step simply by dipping the substrate into an aqueous solution or rolling a droplet across the array (Fig. 1). The rolling droplet method is according to the invention and is useful for printing precious reagents because many spots can be filled without using a large amount of solution. Due to the extreme difference between the superhydrophilicity of the spots and the superhydrophobicity of the barriers, water is easily removed from the barriers but fills the superhydrophilic regions. Examples of arrays of water microdroplets with different geometries and sizes are depicted in Fig. 2A (cf. Fig. 3 for droplet formation using a methyl green solution). Formation of droplets with sharp-edged geometries, such as triangles, is also possible. As can be seen in the images, as soon as the nanoporous superhydrophilic areas are wetted, the underlying polymer becomes transparent due to reduced light scattering caused by matched refractive indexes, allowing easier discrimination of spots and facilitating the use of inverted microscopes. Each microdroplet functions as a small lens with the surface curvature determined by the geometry of the hydrophilic spots (Fig. 2b).

The formation of isolated droplets is reproducible and leads to homogeneous microdroplets. The volumes of the formed droplets depend on the size and geometry of the hydrophilic spots. The smallest microdroplets that could be produced had a volume of 200 pl using a 100 x 100 µm² square micropattern. This was mainly due to the limitations of the resolution using this patterning technique and the inability to measure such small volumes before evaporation occurs. The volume of large droplets is more difficult to control because of the increasingly important role of gravity. This effect was observed for 3 mm × 3 mm square patterns and larger.

### Example 2:

### Deposition of compounds, particles or cells present in an aqueous solution

The rapid formation of droplet arrays on the porous substrate can be used as a convenient tool for the deposition of chemicals, particles, cells, or any other components present in an aqueous solution. Fig. 2C shows an array of microdroplets containing rat mammary carcinoma cells stably expressing enhanced green fluorescent protein (MTly-CMV-eGFP-neo) immediately after droplet formation. This type of an array combines the advantages of microplates where cells present in individual wells are physically isolated from each other, thereby reducing the problem of cross-contamination, with the advantages of miniaturization and parallelization of cell microarrays. However, contrary to the cell microarray method, the diffusion of small molecules in the case of the DropletArray will be confined to the individual microdroplets, which in turn can save a lot of precious materials and avoid cross-contamination. This one-step method for making arrays of thousands of microdroplets incorporating cells or other biological species opens a way to a variety of screening applications.

As in the case of cell microarrays, the array of hydrophilic spots can be pre-printed with libraries of small molecules or transfection reagents. The hydrophobic barriers allow different volumes of solution to be printed per spot, while still confining the solution within the defined hydrophilic area. Thus, volume and concentration adjustments can be made without changing the footprint of the printed spot. The formation of cell-encapsulated droplets on the hydrophilic spots will then be affected by the pre-printed solutions and can then be screened. Presumably, non-adherent cells could also be transfected while being cultured in the droplets.

### Example 3:

### Formation of hydrogel micropads

Culturing cells in 3D microenvironments, such as hydrogels or extracellular matrices, versus 2D cell culture has been shown to more closely mimic the in vivo situation. It was also shown that cell behavior in a 3D microenvironment can be different from the behavior of cells cultured on flat surfaces. Therefore, HT screening of cells in 3D systems is an important challenge yet to be fully realized. The applicability of the DropletArray to perform HT screenings of cells in hydrogel microenvironments is demonstrated herein. Figure 4A schematically shows the process of formation of an array of hydrogel micropads. In the first step, a thiol-functionalized poly(ethylene glycol) crosslinker (PEG-Link) is pre-printed into the superhydrophilic microspots by rolling a droplet across the surface, and then allowed to dry. In the next step, a solution of maleimide-functionalized polyvinyl alcohol (MI-PVA) containing cells is printed in the spots containing crosslinker using the rolling droplet method to create an array of microdroplets. The thiol groups in the PEG-Link form stable thioether bonds with the maleimide groups in the MI-PVA and crosslink to form a stable, biocompatible hydrogel in which living and non-adherent cells can be trapped. Thus, the diffusion of the PEG-Link into the microdroplets from the porous matrix led to the crosslinking of the PVA polymer and formation of a hydrogel within several minutes (Fig. 4). After the hydrogel is formed, the array of hydrogel micropads can be immersed in cell culture medium. Figure 4B shows an array of hydrogel micropads in air which have been incorporated with Rhodamine 6G dye for visibility. In addition, live cells (MTly-CMV-eGFP-neo and mouse neural stem cells) were encapsulated in the hydrogel micropads and cultured for several days without detectable cytotoxicity.

### Example 4:

### Formation of an array of zebrafish eggs

The applicability of the DropletArray is not limited to the highly parallelized immobilization of small biological specimens like single cells. Even by manual preparation, the strictly confined droplets themselves can act as a convenient trap for bigger species that cannot be deposited automatically by methods known in the art. A droplet confined in a 3 mm diameter circle pattern can contain up to 10 µl of liquid when manually filled with solution. Depositing zebrafish embryos, with a diameter of about 1 mm, in droplets of medium results in an array of living, easy-to-observe fish embryos. In this manner, one, two, and even three zebrafish embryos can be captured in one droplet. Using a pipette, the medium can be easily exchanged, thus long cultivation times are possible. As each droplet is its own, enclosed system, drugs added to each droplet do not influence the adjacent zebrafish. Thus, substance screenings on the phenotypic development of zebrafish could easily be carried out. The small droplet volume greatly lowers the amount of biological active compound required for such experiments, while the strict positioning of each egg or embryo within a small area decreases the mobility of the egg or embryo and eases orientation on the grid. For additional immobilization, this approach can be combined with the hydrogel formation and thus create arrays of completely immobilized zebrafish embryos, which greatly eases microscopic observation of detailed areas in the zebrafish.

## Claims

1. A method of forming an array of separated homogenous aqueous fluid microdroplets of a desired shape and size in a desired spatial pattern, comprising the steps of:
(a) providing a patterned substrate with a solid support coated with a porous polymer layer or film comprising
(i) superhydrophilic areas having static, advancing and receding water contact angles (WCAs) of less than 20°, and having the desired shape and size; surrounded by
(ii) superhydrophobic areas having a WCA of greater than 130° and a width of 200 µm or less, and separating the superhydrophilic areas into the desired spatial pattern;
and
(b) applying the aqueous fluid to a multitude of the superhydrophilic areas at the same time, wherein step (b) comprises spreading or rolling an amount of the aqueous fluid along the surface of the patterned substrate.

2. The method of claim 1, wherein step (b) is performed manually.

3. The method of claim 1 or claim 2, wherein the aqueous fluid is selected from the group consisting of water, aqueous solutions, aqueous media, and solutions that can form a hydrogel upon crosslinking or UV-initiated polymerization.

4. The method of any one of claims 1 to 3, wherein the aqueous fluid contains biological specimens.

5. The method of claim 4, wherein the biological specimens are selected from the group consisting of cells, bacteria, viruses and multicellular organisms.

6. The method of claim 5, wherein the multicellular organisms are selected from the group consisting of Danio rerio (zebrafish) and Caenorhabditis elegans.

7. The method of any one of claims 1 to 6, wherein the patterned substrate is a microarray.

8. The method of any one of claims 1 to 7, wherein the patterned substrate has a flat surface.

9. The method of any one of claims 1 to 8, wherein the porous polymer layer or film is biocompatible.

10. The method of any one of claims 1 to 9, wherein the patterned substrate does not comprise physical barriers separating the individual superhydrophilic areas from each other.

11. The method of any of claims 1 to 10, wherein the superhydrophilic areas have at least one compound absorbed thereto which can diffuse into the aqueous fluid microdroplets formed.

12. The method of claim 11, wherein the at least one compound is selected from the group consisting of chemical compounds, pharmaceutically active agents, biologically active agents, toxins, nucleic acids, peptides, proteins, enzymes, crosslinkers, buffer substances, water-soluble polymers and mixtures thereof.

13. The method of any one of claims 1 to 12, wherein the superhydrophilic areas have a crosslinker adhered thereto, and wherein the aqueous fluid is a solution that can form a hydrogel upon crosslinking, so that the aqueous fluid microdroplets form hydrogel micropads.

## Patentansprüche

1. Verfahren für das Erzeugen eines Arrays von getrennten homogenen wässrigen Fluid-Mikrotröpfchen einer gewünschten Form und Größe in einem gewünschten räumlichen Muster, umfassend die Schritte von:
(a) Bereitstellen eines gemusterten Substrats mit einem festen Träger, der mit einer porösen Polymerschicht oder -folie beschichtet ist, umfassend
(i) superhydrophile Bereiche mit statischen, vor- und zurückgehenden Wasserkontaktwinkeln (WCAs) von weniger als 20° und mit der gewünschten Form und Größe; umgeben von
(ii) superhydrophoben Bereichen mit einem WCA von mehr als 130° und einer Breite von 200 µm oder weniger, welche die superhydrophilen Bereiche in das gewünschte räumliche Muster trennen;
und
(b) gleichzeitiges Aufbringen des wässrigen Fluids auf eine Vielzahl der superhydrophilen Bereiche, wobei Schritt (b) das Ausbreiten oder Rollen einer Menge des wässrigen Fluids entlang der Oberfläche des gemusterten Substrats umfasst.

2. Verfahren nach Anspruch 1, wobei Schritt (b) manuell durchgeführt wird.

3. Verfahren nach Anspruch 1 oder Anspruch 2, wobei das wässrige Fluid ausgewählt ist aus der Gruppe, bestehend aus Wasser, wässrigen Lösungen, wässrigen Medien und Lösungen, die bei Vernetzung oder UV-initiierter Polymerisation ein Hydrogel bilden können.

4. Verfahren nach einem der Ansprüche 1 bis 3, wobei die wässrige Fluid biologische Proben enthält.

5. Verfahren nach Anspruch 4, wobei die biologischen Proben ausgewählt sind aus der Gruppe, bestehend aus Zellen, Bakterien, Viren und multizellulären Organismen.

6. Verfahren nach Anspruch 5, wobei die multizellulären Organismen ausgewählt sind aus der Gruppe, bestehend aus *Danio rerio* (Zebrafisch) und *Caenorhabditis elegans.*

7. Verfahren nach einem der Ansprüche 1 bis 6, wobei das gemusterte Substrat ein Mikroarray ist.

8. Verfahren nach einem der Ansprüche 1 bis 7, wobei das gemusterte Substrat eine flache Oberfläche aufweist.

9. Verfahren nach einem der Ansprüche 1 bis 8, wobei die poröse Polymerschicht oder -folie biokompatibel ist.

10. Verfahren nach einem der Ansprüche 1 bis 9, wobei das gemusterte Substrat keine physikalischen Barrieren umfasst, welche die einzelnen superhydrophilen Bereiche voneinander trennen.

11. Verfahren nach einem der Ansprüche 1 bis 10, wobei die superhydrophilen Bereiche mindestens eine daran absorbierte Verbindung aufweisen, die in die erzeugten Mikrotröpfchen des wässrigen Fluids diffundieren kann.

12. Verfahren nach Anspruch 11, wobei die mindestens eine Verbindung ausgewählt ist aus der Gruppe, bestehend aus chemischen Verbindungen, pharmazeutisch aktiven Mitteln, biologisch aktiven Mitteln, Toxinen, Nukleinsäuren, Peptiden, Proteinen, Enzymen, Vernetzern, Puffersubstanzen, wasserlöslichen Polymeren und Mischungen davon.

13. Verfahren nach einem der Ansprüche 1 bis 12, wobei die superhydrophilen Bereiche einen daran angehafteten Vernetzer aufweisen und wobei das wässrige Fluid eine Lösung ist, die beim Vernetzen ein Hydrogel bilden kann, so dass die Mikrotröpfchen des wässrigen Fluids Hydrogel-Mikropads bilden.

## Revendications

1. Procédé de formation d'un réseau de microgouttelettes de fluide aqueux homogènes et séparées d'une forme et d'une taille souhaitées selon un motif spatial souhaité, comprenant les étapes suivantes :
(a) la fourniture à un substrat à motif d'un support solide revêtu d'une couche ou d'un film en polymère poreux comprenant
(i) des zones superhydrophiles ayant des angles de contact avec l'eau (WCA) statiques, rentrant et sortant inférieurs à 20°, et ayant la forme et la taille souhaitées ; entourées
(ii) de zones superhydrophobes ayant un WCA supérieur à 130° et une largeur de 200 *µ*m ou moins, et séparant les zones superhydrophiles en le motif spatial souhaité ;
et
(b) application du fluide aqueux à une multitude des zones superhydrophiles en même temps, dans lequel l'étape (b) comprend l'étalement ou le roulement d'une quantité du fluide aqueux le long de la surface du substrat à motif.

2. Procédé selon la revendication 1, dans lequel l'étape (b) est réalisée manuellement.

3. Procédé selon la revendication 1 ou la revendication 2, dans lequel le fluide aqueux est choisi dans le groupe constitué par l'eau, des solutions aqueuses, des milieux aqueux, et des solutions qui peuvent former un hydrogel lors d'une réticulation ou d'une polymérisation provoquée par UV.

4. Procédé selon l'une quelconque des revendications 1 à 3, dans lequel le fluide aqueux contient des échantillons biologiques.

5. Procédé selon la revendication 4, dans lequel les échantillons biologiques sont choisis dans le groupe constitué par des cellules, des bactéries, des virus et des organismes multicellulaires.

6. Procédé selon la revendication 5, dans lequel les organismes multicellulaires sont choisis dans le groupe constitué par Danio rerio (dard-perche) et Caenorhabditis elegans.

7. Procédé selon l'une quelconque des revendications 1 à 6, dans lequel le substrat à motif est un microréseau.

8. Procédé selon l'une quelconque des revendications 1 à 7, dans lequel le substrat à motif comporte une surface plate.

9. Procédé selon l'une quelconque des revendications 1 à 8, dans lequel la couche ou le film en polymère poreux est biocompatible.

10. Procédé selon l'une quelconque des revendications 1 à 9, dans lequel le substrat à motif ne comprend pas de barrières physiques séparant les zones superhydrophiles individuelles les unes des autres.

11. Procédé selon l'une quelconque des revendications 1 à 10, dans lequel les zones superhydrophiles comportent au moins un composé absorbé sur celles-ci qui peut diffuser dans les microgouttelettes de fluide aqueux formées.

12. Procédé selon la revendication 11, dans lequel l'au moins un composé est choisi dans le groupe constitué par des composés chimiques, des agents pharmaceutiquement actifs, des agents biologiquement actifs, des toxines, des acides nucléiques, des peptides, des protéines, des enzymes, des agents de réticulation, des substances tampons, des polymères solubles dans l'eau et des mélanges de ceux-ci.

13. Procédé selon l'une quelconque des revendications 1 à 12, dans lequel les zones superhydrophiles ont un agent de réticulation qui adhère à celles-ci, et dans lequel le fluide aqueux est une solution qui peut former un hydrogel lors d'une réticulation, de sorte que les microgouttelettes de fluide aqueux forment des microplaquettes en hydrogel.
